# EUROPÄISCHE PATENTSCHRIFT

(11) **EP 3 980 583 B1**
(45) Veröffentlichungstag und Bekanntmachung des Hinweises auf die Patenterteilung: **10.05.2023**
(21) Anmeldenummer: 20729057.8
(22) Anmeldetag: 28.05.2020
(51) Int. Cl.: C25C 3/22, C25C 3/06, C25C 3/12

(54) **VERFAHREN UND ANLAGENVERBUND ZUR BEHANDLUNG DER BEI DER HERSTELLUNG VON ALUMINIUM ANFALLENDEN KOHLENSTOFFOXIDE**
METHOD AND PLANT COMBINATION FOR THE TREATMENT OF CARBON OXIDES PRODUCED DURING THE PRODUCTION OF ALUMINIUM
PROCÉDÉ ET ENSEMBLE D'INSTALLATIONS PERMETTANT DE TRAITER LES OXYDES DE CARBONE OBTENUS LORS DE LA FABRICATION D'ALUMINIUM

(30) Priorität: 05.06.2019 EP 19178470
(43) Veröffentlichungstag der Anmeldung: 13.04.2022
(73) Patentinhaber: BASF SE, 67056 Ludwigshafen am Rhein (DE); thyssenkrupp AG, 45143 Essen (DE); thyssenkrupp Industrial Solutions AG, 45143 Essen (DE)
(72) Erfinder: SCHEIFF, Frederik, 67056 Ludwigshafen (DE); LEDUC, Marc, 67059 Ludwigshafen (DE); BODE, Andreas, 67056 Ludwigshafen (DE); BUEKER, Karsten, 44137 Dortmund (DE); ANTWEILER, Nicolai, 45147 Essen (DE)
(74) Vertreter: BASF IP Association
(86) Internationale Anmeldenummer: PCT/EP2020/064778
(87) Internationale Veröffentlichungsnummer: WO 2020/245015

(56) Entgegenhaltungen:
- DE-A1-102013 102 969
- DE-C1- 19 845 258
- RU-C1- 2 625 152
- US-A- 3 284 334

## Beschreibung

Die vorliegende Erfindung betrifft ein Verfahren zur Behandlung eines in einer Anlage zur Herstellung von Aluminium durch elektrolytische Reduktion von Aluminiumoxid in der Schmelze, unter Verwendung wenigstens einer Anode aus einem kohlenstoffhaltigen Material, anfallenden Abgasstroms, welcher aufgrund der Reduktion des Aluminiumoxids mittels des Kohlenstoffes Kohlenstoffoxide enthält. Gegenstand der vorliegenden Erfindung ist außerdem ein Anlagenverbund umfassend eine Elektrolysevorrichtung zur Herstellung von Aluminium durch schmelzelektrolytische Reduktion von Aluminiumoxid, wenigstens eine Vorrichtung zur Wärmeübertragung, in der wenigstens ein erster Teilstrom des Kohlenstoffoxide enthaltenden Abgasstroms aus der Anlage zur Herstellung von Aluminium auf eine geringere Temperatur abgekühlt wird, sowie wenigstens eine Einrichtung zur Aufreinigung und/oder Konditionierung des Abgasstroms aus der Anlage zur Herstellung von Aluminium.

### Stand der Technik

Die Herstellung von Aluminium erfolgt überwiegend über die Schmelzflusselektrolyse nach dem Hall-Heroult-Verfahren. Bei diesem Verfahren wird ein eutektisches Gemisch aus dem niedrig schmelzenden Aluminiummineral Kryolith (Na₃[AlF₆]) und dem hoch schmelzenden Aluminiumoxid (Korund) der Schmelzflusselektrolyse unterworfen, wobei das Aluminiumoxid reduziert wird. In der Schmelze liegt Aluminiumoxid in seine Ionen dissoziiert vor.

Al₂O₃ → 2 Al³⁺ + 3 O²⁻

Die in der Schmelze befindlichen Aluminiumionen wandern zur Kathode, wo sie Elektronen aufnehmen und zu Aluminiumatomen reduziert werden.

Al³⁺ + 3 e⁻ → Al

Die negativen Sauerstoffionen O²⁻ wandern zur Anode, geben überschüssige Elektronen ab und reagieren mit dem Kohlenstoff der Anode zu Kohlenstoffmonoxid und Kohlenstoffdioxid, die als Gase entweichen.

C + 2 O²⁻ → CO₂ + 4 e⁻

Die gesamte Reaktionsgleichung für den Hall-Heroult-Prozess lautet somit wie folgt:

2 Al₂O₃ + 3 C → 4 Al + 3 CO₂ (1)

Bei der Reduktion von Aluminiumoxid zu Aluminium fallen große Mengen Kohlenstoffdioxid (CO₂) und Kohlenstoffmonoxid (CO) an. Neben diesen beiden Gasen werden Schwefeldioxid (SO₂) und Fluorwasserstoff (HF) emittiert. Kohlenstofftetrafluorid (CF₄), Hexafluorethan (C₂F₆), Schwefelhexafluorid (SF₆) und Siliziumtetrafluorid (SiF₄) sind bei geringen Sauerstoffkonzentrationen mengenmäßig ebenfalls relevant. Die Komponenten CO₂, CO und SO₂ resultieren aus dem Anodenabbrand. Der eingesetzte kalzinierte Petrolkoks aus der Verarbeitung von Rohöl zu Kraftstoffen enthält Schwefelanteile, je nach Qualität im Bereich von beispielsweise 1 bis 7 Gew.-%. In vielen Fällen werden die Abgase der Aluminiumerzeugung in die Atmosphäre abgegeben [Aarhaug et al, "Aluminium Primary Production Off-Gas Composition and Emissions: An Overview", JOM, Vol. 71, No. 9, 2019]. Bei den Emissionen von SO₂ und HF dürfen bestimmte zulässige Grenzwerte nicht überschritten werden. Außerdem werden die Emissionen klimaschädlicher Gase zunehmend reglementiert. Ca. 7 % des weltweiten industriellen Energieverbrauchs und 2,5 % der anthropogenen Treibhausgase sind auf die Aluminiumherstellung zurückzuführen. Im Lebenszyklus der Primäraluminiumherstellung können bis zu 20 kg CO₂-Äquivalente/kg Aluminium entstehen. Die CO₂-Emissionen beliefen sich in Deutschland im Jahr 2018 auf ca. 1 Millionen Tonnen Kohlendioxid-Äquivalente (Treibhausgasemissionen 2018 (VET_Bericht 2018)). Perfluorierte Kohlenwasserstoffe (PFKs) entstehen durch eine erhöhte Spannung, die bei einem zu geringen Anteil an gelöstem Aluminiumoxid (Al₂O₃) auftritt. Strategien zur Reduktion der Emissionen des Hall-Heroult-Prozesses zur Herstellung von Aluminium sind daher von großem wirtschaftlichem und ökologischem Interesse.

In der Literatur sind Untersuchungen zur Abtrennung und Nutzung des Kohlenstoffdioxids zu finden, welches in dem Abgasstrom der Schmelzflusselektrolyse des Aluminiumoxids enthalten ist. Kritisch im Hinblick auf die Wirtschaftlichkeit einer solchen Nutzung sind jedoch die vergleichsweise geringen Kohlenstoffdioxid-Konzentrationen im Abgasstrom. Der Abgasstrom setzt sich aus den Abgasen der Schmelzflusselektrolyse und der Umgebungsluft zusammen. Eine bekannte Strategie zur Aufkonzentrierung ist die Reduktion der Zellenbelüftung, welche zwar zu einer höheren CO₂-Konzentration führt, aber auch eine höhere Zellen- und Abgastemperatur verursacht.

In der EP 2 660 358 A2 wird ein Verfahren zur elektrolytischen Herstellung von Aluminium aus Aluminiumoxid nach dem Hall-Heroult-Prozess beschrieben, bei dem in der Elektrolysezelle anfallende Staubpartikel sowie Abgase, welche insbesondere Fluorwasserstoff, Schwefeldioxid und Kohlenstoffdioxid enthalten, über einen Absaugkanal abgesaugt und in eine Gasbehandlungsvorrichtung geleitet werden. Dort werden die Abgase mit einem Absorbens in Form von Aluminiumoxid in Kontakt gebracht, welches mit Fluorwasserstoff und Schwefeldioxid reagiert, wobei die dabei gebildeten Partikel mittels einer Filtereinrichtung abgetrennt werden. Mit den Abgasen mitgerissene Staubpartikel werden dabei ebenfalls abgetrennt. Verbleibendes Schwefeldioxid kann anschließend noch in einer Waschvorrichtung mittels Seewasser oder Kalk abgetrennt werden. Kohlendioxid kann ebenfalls durch einen Waschvorgang abgetrennt werden, mittels einer Ammoniumcarbonat-Lösung. Abgetrenntes Kohlenstoffdioxid wird bei diesem bekannten Verfahren entsorgt und das gereinigte Abgas wird an die Umgebung abgegeben. Zur Abkühlung des Abgasstroms aus der Elektrolysevorrichtung kann ein Wärmeüberträger verwendet werden, wobei als Kühlmedium Umgebungsluft oder Kühlwasser aus einem Gewässer verwendet wird. Ein Teilstrom der so abgekühlten Abgase kann in die Elektrolysezelle zurückgeführt werden.

Die EP 2 360 296 A1 beschreibt einen ähnlichen Stand der Technik wie das zuvor genannte Dokument. Es wird ein Verfahren zur elektrolytischen Herstellung von Aluminium beschrieben, bei dem Abgase aus der Elektrolyse abgesaugt, von Staub und schädlichen Gasen befreit und gekühlt werden, wobei nach Reinigung und Abkühlung ein Teilstrom der gereinigten und abgekühlten Abgase in die Elektrolysezelle zurückgeführt wird. Bei diesem bekannten Verfahren ist jedoch nicht vorgesehen, in den Abgasen der Elektrolysezelle enthaltene Kohlenstoffoxide einer Verwertung zuzuführen, in dem Sinne, dass diese Gase als Edukte für eine anschließende Synthese chemischer Wertstoffe dienen. Vielmehr wird Kohlenstoffdioxid als ein zu entsorgendes Abfallprodukt angesehen, welches nach Komprimierung in einer stillgelegten Mine deponiert wird.

In der DE 197 57 148 A1 wird ebenfalls ein Verfahren zur Herstellung von Aluminium durch Schmelzelektrolyse aus Aluminiumoxid beschrieben, bei dem staubförmige Bestandteile und Fluorwasserstoff aus dem Abgas mittels eines Gettermaterials entfernt werden. Dabei entsteht Aluminiumfluorid, welches in die Schmelze zurückgeführt werden kann. Eine Aufarbeitung der ebenfalls im Abgas aus der Schmelzelektrolyse enthaltenen Kohlenstoffoxide wird in diesem Dokument nicht beschrieben.

Die Aufgabe der vorliegenden Erfindung besteht darin, ein Verfahren bzw. einen Anlagenverbund der eingangs genannten Gattung zur Verfügung zu stellen, bei dem die Möglichkeit geschaffen wird, bei der elektrolytischen Herstellung von Aluminium anfallende Kohlenstoffoxide wenigstens teilweise einer wirtschaftlich sinnvollen Verwertung zuzuführen.

Eine weitere Aufgabe war, die bei der Anodenherstellung anfallenden Abgase einer sinnvollen Nutzung zuzuführen.

Die Lösung der vorgenannten Aufgabe liefert ein Verfahren der eingangs genannten Art mit den Merkmalen des Anspruchs 1 bzw. ein Anlagenverbund mit den Merkmalen des Anspruchs 16.

Erfindungsgemäß wird wenigstens ein Teilstrom der im Abgasstrom enthaltenen Kohlenstoffoxide gereinigt und/oder konditioniert und mit Wasserstoff umgesetzt und zu Kohlenstoffmonoxid und/oder Methan reduziert wird oder mit einem Wasserstoffstrom gemischt und nachfolgend einer Verwertung in einer chemischen oder biotechnologischen Reaktion zugeführt.

Im Rahmen einer bevorzugten Weiterbildung des erfindungsgemäßen Verfahrens bestehen hier insbesondere drei alternative Möglichkeiten. Gemäß einer ersten Variante können die im Abgasstrom enthaltenen Kohlenstoffoxide einer Einrichtung zugeführt werden, in der eine reverse Wassergas-Shift-Reaktion durchgeführt wird, bei der mindestens ein Anteil des Kohlenstoffdioxids mit Wasserstoff umgesetzt und zu Kohlenstoffmonoxid reduziert und so ein Synthesegasstrom erzeugt wird.

Unter "Synthesegas" im engeren Sinne versteht man industriell hergestellte Gasgemische, die Wasserstoff und Kohlenstoffmonoxid neben weiteren Gasen enthalten. Je nachdem, in welchem Verhältnis Wasserstoff und Kohlenstoffmonoxid in dem Gasgemisch enthalten sind, können aus Synthesegas verschiedene Produkte hergestellt werden, beispielsweise flüssige Kraftstoffe nach dem Fischer-Tropsch-Verfahren bei einem Verhältnis Wasserstoff zu Kohlenstoffmonoxid von 1 - 2 : 1 , Alkohole wie Methanol oder Ethanol bei einem Verhältnis von etwa 2 : 1, oder Methan oder synthetisches Erdgas (SNG) durch Methanisierungsreaktion bei einem Verhältnis von etwa 3 : 1.

Die sogenannte Wassergas-Shift-Reaktion wird gewöhnlich dazu verwendet, den Kohlenstoffmonoxid-Anteil in Synthesegas zu verringern und weiteren Wasserstoff zu produzieren. Dies geschieht gemäß der nachfolgenden Reaktionsgleichung:

CO + H₂O → CO₂ + H₂ (2)

Bei der vorgenannten Reaktion (2) handelt es sich um eine Gleichgewichtsreaktion, die bei geänderten Reaktionsbedingungen, beispielsweise bei Temperaturerhöhung, in umgekehrter Richtung abläuft. Diese Umkehrreaktion wird hierin als reverse Wassergas-Shift-Reaktion bezeichnet und entspricht der nachfolgend wiedergegebenen Reaktionsgleichung:

CO₂ + H₂ → CO + H₂O (3)

Im Rahmen einer bevorzugten Weiterbildung des erfindungsgemäßen Verfahrens kann somit die vorgenannte Reaktion (3) dazu genutzt werden, einen Anteil des bei der Schmelzflusselektrolyse des Aluminiumoxids entstehenden Kohlenstoffdioxids mit Hilfe von Wasserstoff, welcher beispielsweise durch Pyrolyse von Kohlenwasserstoffen gewonnen wird oder aus einer anderen Quelle stammt, in Kohlenstoffmonoxid umzuwandeln, um auf diese Weise weiteres Kohlenstoffmonoxid zu erzeugen und ein Synthesegas zur Verfügung zu stellen, welches einen höheren Anteil an Kohlenstoffmonoxid aufweist, bei gleichzeitig reduziertem Gehalt an Kohlenstoffdioxid, so dass dieses Synthesegasgemisch eine für spezifische weitere Umsetzungen besonders geeignete Zusammensetzung aufweist.

Wenn beispielsweise das Verhältnis Kohlenstoffmonoxid zu Kohlenstoffdioxid in dem Synthesegasgemisch verhältnismäßig groß ist, kann das Synthesegasgemisch gemäß einer bevorzugten Variante der vorliegenden Erfindung beispielsweise zusammen mit Wasserstoff in einer chemischen oder biotechnologischen Anlage genutzt werden.

Eine zweite bevorzugte Variante des erfindungsgemäßen Verfahrens sieht vor, dass in einer Einrichtung eine Sabatier-Reaktion durchgeführt wird, in der Kohlenstoffdioxid und/oder Kohlenstoffmonoxid durch Umsetzung mit Wasserstoff in Methan umgewandelt wird. Die Umsetzung von Kohlenstoffmonoxid mit Wasserstoff folgt gemäß dieser nach dem französischen Chemiker Paul Sabatier benannten Reaktion der nachstehend wiedergegebenen Reaktionsgleichung:

CO + 3 H₂ → CH₄ + H₂O (4)

In ähnlicher Weise kann auch Kohlenstoffdioxid mit Wasserstoff umgesetzt werden gemäß der nachstehend wiedergegebenen Reaktionsgleichung:

CO₂ + 4 H₂ → CH₄ + 2 H₂O (5)

Das auf diese Weise gewonnene Methan kann entweder als Energieträger dienen und beispielsweise gespeichert werden oder aber als Edukt in einer chemischen oder biotechnologischen Anlage für die Synthese anderer chemischer Wertprodukte genutzt werden.

Gemäß einer dritten bevorzugten Variante der Erfindung werden die im Abgasstrom enthaltenen Kohlenstoffoxide einer Einrichtung zugeführt, in der sie mit einem Wasserstoffstrom gemischt werden. Ein solches Gemisch enthält dann beispielsweise Kohlenstoffmonoxid und Wasserstoff und bildet ebenfalls ein Synthesegas, welches als Einsatzgasstrom in einer chemischen oder biotechnologischen Anlage genutzt werden kann.

Eine bevorzugte Weiterbildung des erfindungsgemäßen Verfahrens sieht vor, dass mindestens ein Teilstrom von Abgasen aus der Anlage zur Herstellung von Aluminium zunächst in einer ersten Einrichtung zur Aufreinigung und/oder Konditionierung des Abgases behandelt wird, bevor der Abgasstrom der Einrichtung zugeführt wird, in der die reverse Wassergas-Shift-Reaktion oder die Sabatier-Reaktion durchgeführt oder das Abgas mit Wasserstoff gemischt wird. In einer solchen Einrichtung kann beispielsweise die Entfernung für die weitere Umsetzung hinderlicher bzw. umweltschädlicher Gasbestandteile des Abgases aus der Anlage zur Herstellung von Aluminium erfolgen, beispielsweise von Fluorwasserstoff oder Schwefeldioxid. In dieser Einrichtung können beispielsweise gasförmige Bestandteile aus dem Abgas herausgewaschen werden oder Feststoffpartikel durch Filtration oder Adsorption entfernt werden. In dieser Einrichtung können aber auch beispielsweise Gase zugemischt werden, wenn eine Variierung der Zusammensetzung des Abgasgemisches vorteilhaft ist für den nachfolgenden Umsetzungsprozess zur Herstellung chemischer Wertprodukte.

Gemäß einer ersten Möglichkeit im Rahmen einer bevorzugten Weiterbildung der Erfindung wird mindestens ein Teilstrom, beispielsweise ein zweiter Teilstrom des Abgasstroms nach Verlassen der Anlage zur Herstellung von Aluminium zunächst in einer Vorrichtung zur Wärmeübertragung auf eine geringere Temperatur abgekühlt und erst danach der vorgenannten Einrichtung zur Aufreinigung und/oder Konditionierung des Abgases zugeführt. Diese Abkühlung kann zum Beispiel zur Wärmeübertragung in einem Wärmeüberträger erfolgen, so dass die in den heißen Abgasen enthaltene Energie beispielsweise in anderen Bereichen der Anlage zur Erwärmung eines Stoffstroms genutzt werden kann.

Alternativ dazu oder auch zusätzlich kann auch mindestens ein-im Falle der zuvor beschriebenen Variante gegebenenfalls erster--Teilstrom der Abgase aus der Anlage zur Herstellung von Aluminium ohne vorherige Abkühlung der Einrichtung zur Aufreinigung und/oder Konditionierung des Abgases zugeführt werden. Man kann den Abgasstrom somit auch aufteilen und einen Teilstrom des Abgasstroms zunächst abkühlen und einen weiteren Teilstrom des Abgasstroms ungekühlt weiterverwenden. Man kann alternativ auch den gesamten Abgasstrom ungekühlt verwenden oder den gesamten Abgasstrom vor der weiteren Verarbeitung abkühlen.

Eine bevorzugte Weiterbildung der Erfindung sieht vor, dass wenigstens ein erster Teilstrom des Kohlenstoffoxide enthaltenden Abgasstroms aus der Anlage zur Herstellung von Aluminium in diese Anlage zurückgeführt wird.

Dieser Teilstrom des Abgases, der in die Anlage zurückgeführt wird, kann ein zuvor in einer Vorrichtung zur Wärmeübertragung auf eine geringere Temperatur abgekühlter Abgasstrom sein. Diese Maßnahme hat den Vorteil, dass die aus der Umgebungsluft resultierenden Bestandteile des Abgasstromes durch eine Rückführung des Abgasstroms aus der Reduktionszelle substituiert werden und sich somit die beiden Komponenten Kohlenstoffdioxid und Kohlenstoffmonoxid im Abgasstrom der Elektrolysezelle anreichern.

Wesentliche Bestandteile des Abgasstromes aus der Elektrolysezelle bei der Schmelzflusselektrolyse von Aluminiumoxid sind die Komponenten Kohlenstoffdioxid und Kohlenstoffmonoxid, die aus dem Anodenabbrand der aus Kohlenstoff hergestellten Anoden resultieren. Die Anoden bestehen aus kalziniertem Petrolkoks oder Pyrolysekohlenstoff sowie in der Regel Pech als Bindemittel und werden beispielsweise in Schachtöfen oder Drehrohröfen unter Einsatz von Energie gebacken. Die fertigen Anoden werden in der Hall-Heroult-Elektrolyse zur Produktion von Aluminium unter Einsatz von Kryolith und Energie eingesetzt. Die Abgase aus der Reduktionszelle resultieren im Wesentlichen aus der elektrolytischen Reduktion des Aluminiumoxids zu Aluminium gemäß der oben wiedergegebenen Reaktionsgleichung (1), weiterhin der Reoxidierung des Aluminiums gemäß der nachstehend wiedergegebenen Gleichung (6):

2 Al + 3 CO₂ → Al₂O₃ + 3 CO (6)

der Boudouard-Reaktion zwischen primären CO₂-Gasen und Anodenkohlenstoff gemäß der nachstehend wiedergegebenen Reaktionsgleichung (7):

CO₂ + C → 2 CO (7)

und einem signifikanten Anodenabbrand durch den Luftsauerstoff oberhalb des elektrolytischen Bades gemäß der nachfolgend wiedergegebenen Reaktionsgleichung (8):

C + O₂ → CO₂ (8)

Statistisch ergibt sich beispielsweise folgende Aufteilung für den Anodenkohlenstoffverbrauch:

| Mechanismus | Anodenverbrauch, Gew.-% |
|---|---|
| 2 Al₂O₃ + 3 C → 4 Al + 3 CO₂ | 66 - 76 |
| C + O₂ → CO₂ und 2 C + O₂ → 2 CO | 8 - 15 |
| CO₂ + C → 2 CO | 5 - 6 |
| Staub | 0,3 |
| 2 Al + 3 CO₂ → Al₂O₃ + 3 CO | 7 - 8 |
| Pyrolyse | 0,2 |
| Schwefel, Metallverunreinigungen und rezyklierte Anodenrückstände | 3,5 - 4,5 |
| Netto Kohlenstoffverbrauch/ kg C/t Al | 400 - 450 |

Der Anodenabbrand durch Luftsauerstoff verursacht mit einem Kohlenstoffverbrauch von etwa 8 Gew.-% bis etwa 15 Gew.-% einen signifikanten Anteil am Gesamtverbrauch. Des Weiteren ist durch die Verdünnung der anfallenden Abgase mit Umgebungsluft eine Abtrennung der Treibhausgase kostenintensiv. Gemäß der vorliegenden Erfindung wird bevorzugt eine Teilrezylierung des Abgasstromes vorgeschlagen. Die Reaktion von CO₂ und CO mit dem Kohlenstoff der Anode ist bei den vorliegenden Verweilzeiten der Gasphase kinetisch stark limitiert. Die Konsequenz ist ein stark reduzierter Anodenabbrand und eine Aufkonzentrierung der Komponenten CO₂ und CO im Abgasstrom der Reduktionszelle. Hierzu wird bevorzugt der Abgasstrom in einem Wärmeüberträger auf eine geringere Temperatur abgekühlt und teilweise zurückgeführt. Ein Teil des Abgasstromes kann beispielsweise, je nachdem, welche Art der weiteren Verwertung vorgesehen ist, entweder nach der Abkühlung weiterverwendet oder ohne Abkühlung in den nächsten Anlagenteil überführt werden. Je nach Zusammensetzung des Abgasstromes ist dann gegebenenfalls eine Aufreinigung und Konditionierung erforderlich.

Insbesondere bei Schaffung eines Anlagenverbunds umfassend Anlagenbereiche, in denen eine Kohlenwasserstoffpyrolyse, beispielsweise eine Methanpyrolyse zur Anodenherstellung einerseits erfolgt und Anlagenbereiche, in denen die Schmelzflusselektrolyse zur Produktion von Aluminium andererseits erfolgt, können die bei der Aluminiumherstellung anfallenden Kohlenstoffoxide und ggf. die bei der Anodenherstellung entstehenden Abgase in räumlicher Nähe zum Ort Ihrer Entstehung sinnvoll verwertet werden.

Gemäß einer bevorzugten Weiterentwicklung des erfindungsgemäßen Verfahrens wird ein aus dem Abgasstrom der Elektrolysezelle gewonnener Synthesegasstrom vorzugsweise zur Herstellung von Methanol, wenigstens einem Alkohol und/oder wenigstens einem anderen chemischen Wertprodukt verwendet. Unter anderen chemischen Wertprodukten werden organische Verbindungen auf Kohlenstoffbasis quasi beliebiger Art verstanden, die sich aus Synthesegasen herstellen lassen wie zum Beispiel Olefine, Aldehyde, Ether etc., mit Hilfe an sich bekannter Herstellungsverfahren, oder auch Kraftstoffe oder Kraftstoffgemische wie zum Beispiel Benzin oder Diesel oder energiereiche Gase wie zum Beispiel Methan oder andere höhere gasförmige oder flüssige Kohlenwasserstoffe und dergleichen.

Es wurde oben bereits erwähnt, dass der für die reverse Wassergas-Shift-Reaktion oder die Sabatier-Reaktion oder für die Mischung mit den Kohlenstoffoxiden aus dem Abgas zugeführte Wasserstoff beispielsweise durch Pyrolyse von Kohlenwasserstoffen, insbesondere Methan oder Erdgas erzeugt werden kann. Ein weiterer Vorteil ergibt sich bei dieser Variante des Verfahrens, dass der bei der Pyrolyse von Kohlenwasserstoffen, insbesondere Methan oder Erdgas ebenfalls anfallende Pyrolysekohlenstoff zur Herstellung von Anoden für die elektrolytische Herstellung von Aluminium verwendet werden kann. Ein besonderer Vorteil des Pyrolysekohlenstoffs gegenüber konventionellem kalziniertem Petrolkoks besteht darin, dass nahezu kein Schwefel enthalten ist und somit die Schwefelemissionen drastisch reduziert werden.

Wenn man gemäß einer bevorzugten Variante des Verfahrens eine Abkühlung mindestens eines Teilstroms der Abgase vorsieht, dann ergibt sich weiterhin der Vorteil, dass in der Vorrichtung zur Wärmeübertragung durch den Abgasstrom gleichzeitig ein methanhaltiger Gasstrom, insbesondere ein Einsatzgasstrom, den man für die Pyrolyse des Kohlenwasserstoffs, insbesondere von Methan oder Erdgas verwendet, erwärmt werden kann, so dass an dieser Stelle die im Abgas enthaltene Energie im Prozess genutzt werden kann.

Eine mögliche Variante des erfindungsgemäßen Verfahrens sieht vor, dass der nach Aufreinigung und Konditionierung erhaltene Abgasstrom enthaltend Kohlenstoffdioxid und Kohlenstoffmonoxid direkt chemisch verwertet wird. Alternativ dazu wird dem Abgasstrom Wasserstoff zugemischt, bevor eine chemische Verwertung des Gasgemisches erfolgt. Wiederum alternativ dazu kann durch die oben erwähnte reverse Wassergas-Shift-Reaktion zunächst der Kohlenstoffmonoxidanteil erhöht werden und der dann erhaltene Synthesegasstrom in einer chemischen oder biotechnologischen Anlage zu chemischen Produkten wie beispielsweise Methanol, höheren Alkoholen oder anderen chemischen Wertprodukten umgesetzt werden. Gemäß einer weiteren alternativen Variante des Verfahrens kann ein Teil oder das gesamte Kohlenstoffmonoxid und/oder ein Teil oder das gesamte Kohlenstoffdioxid in einer Sabatier-Reaktion in Methan umgewandelt werden.

Gegenstand der vorliegenden Erfindung ist weiterhin ein Anlagenverbund umfassend eine Elektrolysevorrichtung zur Herstellung von Aluminium durch schmelzelektrolytische Reduktion von Aluminiumoxid unter Verwendung wenigstens einer Anode aus einem kohlenstoffhaltigen Material, wenigstens eine Vorrichtung zur Wärmeübertragung, in der wenigstens ein Teilstrom des Kohlenstoffoxide enthaltenden Abgasstroms aus der Anlage zur Herstellung von Aluminium auf eine geringere Temperatur abgekühlt wird, sowie wenigstens eine Einrichtung zur Aufreinigung und/oder Konditionierung des Abgasstroms aus der Anlage zur Herstellung von Aluminium, wobei der Anlagenverbund erfindungsgemäß weiterhin wenigstens einen Reaktor zur Umsetzung des Abgasstroms mit Wasserstoff zu Synthesegas und/oder zu Methan und/oder eine Vorrichtung zum Mischen des Abgasstroms mit Wasserstoff für die nachfolgende Verwertung in eine chemische oder biotechnologische Anlage zur Herstellung von Methanol, wenigstens einem Alkohol und/oder wenigstens einem anderen chemischen Wertprodukt, umfasst.

Ein solches Anlagenkonzept hat den Vorteil, dass man den Abgasstrom aus der Schmelzelektrolyse des Aluminiumoxids in mehrfacher Hinsicht innerhalb eines Verbunds mehrerer Anlagenteile einer Anlage nutzen kann. Zum einen erzeugt man aus in dem Abgas enthaltenen Kohlenstoffoxiden beispielsweise ein Synthesegas oder ein methanhaltiges Gasgemisch, welches sich zur Herstellung chemischer Wertprodukte eignet. Zusätzlich kann die in dem Abgasstrom enthaltene Wärme für eine Wärmeübertragung genutzt werden, bei der ein Einsatzgasstrom für die Pyrolyse von Kohlenwasserstoffen vorgewärmt wird, wobei bei dieser Pyrolyse wiederum Wasserstoff erzeugt wird, der dem Synthesegas zugemischt oder für die Sabatier-Reaktion verwendet werden kann. Außerdem kann innerhalb des Anlagenverbunds noch der Pyrolysekohlenstoff aus der Pyrolyse der Kohlenwasserstoffe bei der Herstellung der Anoden für die Schmelzflusselektrolyse genutzt werden.

Die bei der Herstellung der Anode entstehenden leichtflüchtigen Kohlenwasserstoffen (siehe z.B. Aarhaug et al., "A Study of Anode Baking Gas Composition", Light Metals 2018, pp 1379-1385), insbesondere Methan, Benzol und mehrkernige Aromaten, können vorteilhaft in den Reaktor zur Kohlenwasserstoffpyrolyse zurückgeführt werden. Beispielsweise werden diese leichtflüchtigen Kohlenwasserstoffe über eine Leitung (27) von der Vorrichtung für die Anodenherstellung (1) in den Reaktor zur Kohlenwasserstoffpyrolyse (21) zugeführt oder diese leichtflüchtigen Kohlenwasserstoffe werden über eine Leitung (27) der Zuführleitung (22) für Methan oder andere Kohlenwasserstoffe zum Reaktor zur Kohlenwasserstoffpyrolyse (21) zugemischt.

Die ggf. in dem Anodenabgas vorhandenen perfluorierten Kohlenwasserstoffe, PFKs, werden in der Methanpyrolyse in Fluorwasserstoff umgesetzt. Das Fluorwasserstoff wird vorteilhaft aus dem Gasstrom entfernt, beispielsweise mit Hilfe von Al2O3 bzw. Al(OH)3 adsorbiert/absorbiert. Das Fluorid-beladene Adsorbens wird vorteilhaft in die Kryolith-Schmelze gegeben und das Fluorid damit im Kreis gefahren.

Dabei umfasst der erfindungsgemäße Anlagenverbund bevorzugt weiterhin eine Einrichtung, der die im Abgasstrom enthaltenen Kohlenstoffoxide zugeführt werden, in der eine reverse Wassergas-Shift-Reaktion durchgeführt wird, bei der mindestens ein Anteil des Kohlenstoffdioxids mit Wasserstoff umgesetzt und zu Kohlenstoffmonoxid reduziert und so ein Synthesegasstrom erzeugt wird oder in der eine Sabatier-Reaktion durchgeführt wird, in der Kohlenstoffdioxid und/oder Kohlenstoffmonoxid durch Umsetzung mit Wasserstoff in Methan umgewandelt wird oder in der die im Abgasstrom enthaltenen Kohlenstoffoxide mit einem Wasserstoffstrom gemischt werden.

Außerdem umfasst der erfindungsgemäße Anlagenverbund bevorzugt weiterhin wenigstens zwei voneinander unabhängige Leitungen, wobei mittels der ersten Leitung ein in der Vorrichtung zur Wärmeübertragung abgekühlter erster Abgasstrom einerseits und davon unabhängig mittels der zweiten Leitung ein zweiter ungekühlter Abgasstrom direkt aus der Anlage zur Herstellung von Aluminium der Einrichtung zur Aufreinigung und/oder Konditionierung zugeführt werden können. Diese mögliche konstruktive Variante des erfindungsgemäßen Anlagenverbunds schafft die Möglichkeit, die in dem Abgasstrom enthaltene Wärmeenergie nur teilweise im Wärmetausch für die Erwärmung eines anderen Einsatzgasstroms zu nutzen, während die in dem nicht abgekühlten Teilstrom des Abgases, der gegebenenfalls nach Aufreinigung und Konditionierung direkt zur Erzeugung eines Synthesegasgemisches oder methanhaltigen Gasgemisches verwendet wird, enthaltene Wärmeenergie in dem weiteren Synthese- und Verwertungsprozess genutzt werden kann.

Nachfolgend wird die vorliegende Erfindung anhand von Ausführungsbeispielen unter Bezugnahme auf die beiliegende Zeichnung näher beschrieben. Dabei zeigt:

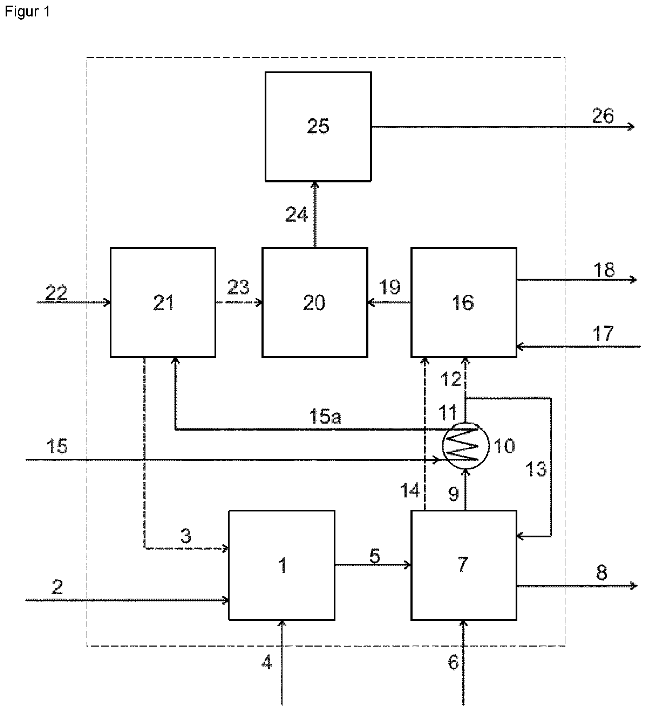

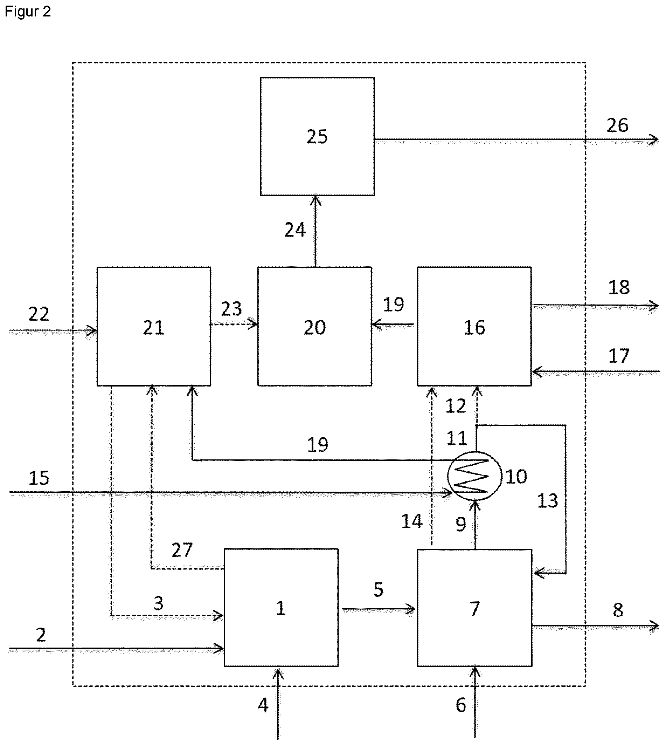

Figur 1 und 2 ein vereinfachtes Anlagenschema einer erfindungsgemäßen Anlage zur Behandlung eines bei der Herstellung von Aluminium durch elektrolytische Reduktion von Aluminiumoxid in der Schmelze anfallenden Abgasstroms.

Nachfolgend wird auf die Figur 1 und 2 Bezug genommen und anhand dieser schematisch vereinfachten Darstellung werden eine beispielhafte Ausführungsvariante des erfindungsgemäßen Verfahrens sowie ein in dem Verfahren verwendbarer Anlagenverbund näher erläutert. Es sind in der Zeichnung nur die wesentlichen Anlagenteile eines solchen Anlagenverbunds beispielhaft dargestellt. Der Anlagenverbund umfasst eine Wasserstoffquelle, insbesondere einen Pyrolysereaktor 21, in dem eine Pyrolyse von Kohlenwasserstoffen, beispielsweise von Methan durchgeführt wird. Dazu wird diesem Pyrolysereaktor 21 oder einer komplexeren Einrichtung umfassend einen solchen Pyrolysereaktor über eine Zuführleitung 15 Methan zugeführt sowie über eine Einrichtung 22 wird dem Reaktor 21 Energie zugeführt, um das Methan auf die für die Pyrolyse notwendige Temperatur von beispielsweise mehr als 800 ° C zu bringen. In dem Pyrolysereaktor 21 entstehen durch die pyrolytische Zersetzung Wasserstoff und Pyrolysekohlenstoff. Der Wasserstoff wird aus dem Reaktor 21 über die Leitung 23 einem weiteren Reaktor 20 zugeführt, indem beispielsweise eine reverse Wassergas-Shift-Reaktion oder eine Sabatier-Reaktion stattfindet, die später noch näher erläutert wird. Der in dem Pyrolysereaktor 21 erzeugte Pyrolysekohlenstoff wird über eine Zuführeinrichtung 3 einer Vorrichtung 1 zugeführt, in der aus dem Pyrolysekohlenstoff Anoden für die Schmelzelektrolyse 7 nach dem Hall-Heroult-Prozess hergestellt werden. Grundsätzlich wäre es möglich, Anoden aus reinem Pyrolysekohlenstoff herzustellen. Vorzugsweise verwendet man jedoch kalzinierte Petrolkoksgemische, mischt dem Petrolkoks Pyrolysekohlenstoff zu und presst dann dieses Gemisch nach Zugabe von Pech zu Anoden, die danach gebacken werden. Die bei der Herstellung der Anode entstehenden leichtflüchtigen Kohlenwasserstoffen werden über eine Leitung 27 in den Pyrolysereaktor 21 zurückgeführt.

Der oben erwähnten Vorrichtung 1, bei der es sich beispielsweise um einen Schachtofen oder Drehrohrofen handeln kann, wird über eine weitere Zuführeinrichtung 2 ein Bindemittel, beispielsweise Pech zugeführt und die auf diese Weise in der Vorrichtung 1 hergestellten Elektroden (Anoden) werden dann über eine weitere Zuführeinrichtung 5 von der Vorrichtung 1 zu der Anlage 7 gefördert, in der die Schmelzflusselektrolyse von Aluminiumoxid erfolgt. Dieser Anlage 7 werden über diverse Zuführeinrichtungen 6, die hier schematisch vereinfacht nur durch eine einfache Linie dargestellt sind, die weiteren Edukte zugeführt, die für die Schmelzflusselektrolyse notwendig sind, nämlich zum einen das Aluminiumoxid, Kryolith, welcher zur Erniedrigung des Schmelzpunktes der aufzuschmelzenden Feststoffe verwendet wird, sowie Energie, die notwendig ist, um dieses Feststoffgemisch auf die Schmelztemperatur des Eutektikums zu bringen, die in der Regel bei etwa 950 ° C liegt. In dieser Anlage 7 entsteht dann als Produkt Aluminium, welches über die Abführeinrichtung 8 aus der Anlage abgeführt werden kann. Weiterhin entsteht durch die Oxidation der aus pyrolytischem Kohlenstoff bestehenden Anoden in der Anlage 7 ein Gasgemisch aus Kohlenstoffdioxid und Kohlenstoffmonoxid in einem Verhältnis, welches von diversen Parametern bei der Elektrolyse des Aluminiumoxids abhängt. Dieses Gasgemisch kann beispielsweise über eine erste Leitung 9 aus der Anlage 7 abgeführt und einem Wärmetauscher 10 zugeführt werden, in dem eine Abkühlung des Gasgemisches erfolgt. Der Wärmetausch erfolgt dabei im Austausch mit dem Methan oder Erdgas, welches über die Leitung 15 zugeführt wird, auf diese Weise vorgewärmt wird und dann über die Leitung 15 a dem Pyrolysereaktor 21 zugeführt wird. Die abgekühlten Abgase gelangen dann in die Leitung 11. Dies kann als ein Beispiel für die Energieintegration in einem erfindungsgemäßen Anlagenverbund angesehen werden, wobei hier auch alternative Möglichkeiten bestehen.

Stromabwärts des Wärmetauschers 10 wird nun der Gasstrom 11 aufgeteilt, wobei ein erster Teilstrom über die gestrichelt dargestellte Leitung 12 der Einrichtung 16 zur Aufreinigung und Konditionierung der Abgase zugeführt wird. Ein zweiter Teilstrom des abgekühlten Abgases wird hingegen über die Leitung 13 zu der Anlage 7, in der die Schmelzflusselektrolyse von Aluminiumoxid stattfindet, zurückgeführt, wodurch sich die Abgase in der Elektrolysezelle mit Kohlenstoffoxiden anreichern.

Nachdem der Abgasstrom in der Einrichtung 16 aufgereinigt und konditioniert wurde, wird das Gasgemisch über die Leitung 19 einem Reaktor 20 zugeführt, in dem eine reverse Wassergas-Shift-Reaktion oder beispielsweise auch eine Sabatier-Reaktion durchgeführt werden kann. Im einfachsten Fall kann es alternativ auch so sein, dass der mit 20 bezeichnete Anlagenteil lediglich eine Mischvorrichtung ist, in der der Gasstrom aus der Leitung 19, der die Kohlenstoffoxide enthält, mit Wasserstoff aus der Leitung 23 gemischt wird.

Die in dem Reaktor 20 beispielsweise durchgeführte reverse Wassergas-Shift-Reaktion, die nach der oben wiedergegebenen Reaktionsgleichung (3) abläuft, dient dazu, den Anteil des Kohlenstoffdioxids in dem Gasgemisch zu senken und den Anteil an Kohlenstoffmonoxid in dem Gasgemisch zu erhöhen. Dazu wird dem Reaktor 20 über die Leitung 23 Wasserstoff zugeführt, welcher mit dem Gasgemisch aus der Anlage 7 zur Schmelzelektrolyse reagiert, wobei dem Reaktor 20 das Kohlenstoffoxide enthaltende Gasgemisch über die Leitung 19 zugeführt wird, die die Einrichtung 16 zur Aufreinigung und Konditionierung der Abgase mit dem Reaktor 20 verbindet. Es entsteht in der Vorrichtung 20 ein Gasgemisch, welches unter anderem Kohlenstoffmonoxid und Wasserstoff enthält und somit als Synthesegas geeignet ist. Dieses Synthesegas wird über die Leitung 24 einer chemischen oder biotechnologischen Anlage 25 zugeführt, in der mittels an sich bekannter Methoden chemische Wertstoffe wie beispielsweise Methanol, höhere Alkohole oder dergleichen synthetisiert werden können. Das so gewonnene Produkt kann über die Leitung 26 aus der Anlage 25 abgeführt werden.

Alternativ dazu kann man in dem Reaktor auch beispielsweise eine Sabatier-Reaktion durchführen, bei der in dem aus der Leitung 19 zugeführten Gasstrom enthaltenes Kohlenstoffdioxid und/oder Kohlenstoffmonoxid mit Wasserstoff zu Methan umgesetzt wird. Dazu verwendet man den Wasserstoff, den man über die Leitung 23 dem Reaktor 20 zuführt. Das so hergestellte Methan kann entweder über die Leitung 24 einer chemischen oder biotechnologischen Anlage 25 zugeführt und dort wie oben beschrieben weiterverarbeitet werden oder auch gegebenenfalls abgeführt und gespeichert werden.

Eine dritte mögliche Alternative besteht darin, dass es sich bei der Anlage 20 um eine einfache Mischvorrichtung handelt, der man den Gasstrom aus der Leitung 19, welcher Kohlenstoffoxide aus dem Abgas enthält und Wasserstoff über die Leitung 23 zuführt, um so ein Gasgemisch zu erhalten, welches sich wiederum für weitere Synthesen von chemischen Wertstoffen wie beispielsweise organischen Verbindungen in der chemischen oder biotechnologischen Anlage 25 eignet.

Eine alternative Variante der Erfindung sieht vor, dass man den Wärmetauscher 10 umgeht und die Abgase aus der Schmelzelektrolyse vollständig oder nur einen Teilstrom davon aus der Anlage 7 über die in Figur 1 gestrichelt dargestellte Leitung 14 direkt und somit ungekühlt in die Einrichtung 16 zur Aufreinigung und Konditionierung einleitet. In dieser Einrichtung können diverse Reinigungsprozesse stattfinden und außerdem können über die Leitung 17 diverse Stoffströme der Einrichtung 16 zugeführt werden, um den Abgasstrom aus der Schmelzelektrolyse 7 zu reinigen, das heißt unerwünschte Bestandteile zu entfernen, beispielsweise durch Waschvorgänge und/oder über Filtervorrichtungen. Weiterhin können über die Leitung 17 auch Stoffströme zugeführt werden, wie beispielsweise zusätzliche Gase, um auf diese Weise die Zusammensetzung des Gasgemisches in der Einrichtung 16 gezielt zu verändern, so dass sich eine geänderte Zusammensetzung ergibt, die zu einem für nachfolgende Umsetzungen und Syntheseschritte in dem Reaktor 20 und/oder in der chemischen oder biotechnologischen Anlage 25 vorteilhaften Gasgemisch führt. Bestandteile, die in der Einrichtung 16 aus dem Abgasstrom entfernt werden, können über die Leitung 18 aus der Einrichtung 16 abgeführt werden.

### Bezugszeichenliste

- 1: Schachtofen oder Drehrohrofen
- 2: Zuführeinrichtung für Pech und gegebenenfalls Petrolkoksen oder anderen Kohlenstoffquellen
- 3: Zuführeinrichtung für Pyrolysekohlenstoff
- 4: Einrichtung für die Energiezufuhr
- 5: Zuführeinrichtung für Anoden
- 6: Einrichtung für die Energiezufuhr
- 7: Schmelzelektrolyse von Aluminiumoxid
- 8: Ausschleusung von Aluminium
- 9: Abgasstrom
- 10: Vorrichtung zur Wärmeübertragung
- 11: Abgasstrom
- 12: Abgasstrom
- 13: zurückgeführter Abgasstrom
- 14: ungekühlter Abgasstrom
- 15: zugeführtes Methan
- 15 a: Leitung für vorgewärmtes Methan
- 16: Einrichtung zur Aufreinigung und Konditionierung
- 17: Input
- 18: Output
- 19: Gasstrom von Kohlenstoffoxiden
- 20: Reverse Wassergas-Shift-Reaktion
- 21: Kohlenwasserstoffpyrolyse, Pyrolysereaktor
- 22: Zuführeinrichtung für Energie
- 23: Leitung für Wasserstoff
- 24: Synthesegasgemisch
- 25: chemische oder biotechnologische Anlage
- 26: Ausgangsleitung für Produkt
- 27: Leitung für leichtflüchtige Kohlenwasserstoffe

## Patentansprüche

1. Verfahren zur Behandlung eines in einer Anlage zur Herstellung von Aluminium durch elektrolytische Reduktion von Aluminiumoxid in der Schmelze, unter Verwendung wenigstens einer Anode aus einem kohlenstoffhaltigen Material, anfallenden Abgasstroms, welcher aufgrund der Reduktion des Aluminiumoxids mittels des Kohlenstoffes Kohlenstoffoxide enthält, **dadurch gekennzeichnet, dass** wenigstens ein Teilstrom der im Abgasstrom enthaltenen Kohlenstoffoxide gereinigt und/oder konditioniert wird, mit Wasserstoff umgesetzt und zu Kohlenstoffmonoxid und/oder Methan reduziert wird oder mit einem Wasserstoffstrom gemischt und nachfolgend einer Verwertung in einer chemischen oder biotechnologischen Reaktion zugeführt wird.

2. Verfahren nach Anspruch 1, **dadurch gekennzeichnet, dass** die im Abgasstrom enthaltenen Kohlenstoffoxide einer Einrichtung (20) zugeführt werden, in der eine reverse Wassergas-Shift-Reaktion durchgeführt wird, bei der mindestens ein Anteil des Kohlenstoffdioxids mit Wasserstoff umgesetzt und zu Kohlenstoffmonoxid reduziert und so ein Synthesegasstrom (24) erzeugt wird.

3. Verfahren nach Anspruch 1, **dadurch gekennzeichnet, dass** die im Abgasstrom enthaltenen Kohlenstoffoxide einer Einrichtung (20) zugeführt werden, in der eine Sabatier-Reaktion durchgeführt wird, in der Kohlenstoffdioxid und/oder Kohlenstoffmonoxid durch Umsetzung mit Wasserstoff in Methan umgewandelt wird.

4. Verfahren nach Anspruch 1, **dadurch gekennzeichnet, dass** die im Abgasstrom enthaltenen Kohlenstoffoxide einer Einrichtung (20) zugeführt werden, in der sie mit einem Wasserstoffstrom (23) gemischt werden.

5. Verfahren nach einem der Ansprüche 1 bis 4, **dadurch gekennzeichnet, dass** mindestens ein Teilstrom (14) von Abgasen aus der Anlage (7) zur Herstellung von Aluminium ohne vorherige Abkühlung der Einrichtung (16) zur Aufreinigung und/oder Konditionierung des Abgases zugeführt wird.

6. Verfahren nach einem der Ansprüche 1 bis 5, **dadurch gekennzeichnet, dass** mindestens ein Teilstrom (12), insbesondere ein zweiter Teilstrom (12) des Abgasstroms (11) nach Verlassen der Anlage zur Herstellung von Aluminium zunächst in einer Vorrichtung (10) zur Wärmeübertragung auf eine geringere Temperatur abgekühlt wird und danach der Einrichtung (16) zur Aufreinigung und/oder Konditionierung des Abgases zugeführt wird.

7. Verfahren nach einem der Ansprüche 1 bis 6, **dadurch gekennzeichnet, dass** wenigstens ein erster Teilstrom (13) des Kohlenstoffoxide enthaltenden Abgasstroms (9) aus der Anlage (7) zur Herstellung von Aluminium in diese Anlage zurückgeführt wird.

8. Verfahren nach Anspruch 7, **dadurch gekennzeichnet, dass** der Abgasstrom (9) nach Verlassen der Anlage zunächst in einer Vorrichtung (10) zur Wärmeübertragung auf eine geringere Temperatur abgekühlt wird und wenigstens ein erster Teilstrom (13) des abgekühlten Abgasstroms (11) in die Anlage (7) zur Herstellung von Aluminium zurückgeführt wird.

9. Verfahren nach einem der Ansprüche 1 bis 8, **dadurch gekennzeichnet, dass** ein die Kohlenstoffoxide enthaltender Abgasstrom (9) aus der Anlage (7) zur Herstellung von Aluminium zunächst in einer Vorrichtung (10) zur Wärmeübertragung auf eine geringere Temperatur abgekühlt wird und dieser abgekühlte Gasstrom danach in mindestens zwei Teilströme (12, 13) aufgeteilt wird, von denen ein Teilstrom (12) der Einrichtung (16) zur Aufreinigung und/oder Konditionierung des Abgases zugeführt wird, während ein weiterer Teilstrom (13) in die Anlage (7) zur Herstellung von Aluminium zurückgeführt wird.

10. Verfahren nach einem der Ansprüche 1 bis 9, **dadurch gekennzeichnet, dass** ein in der Einrichtung (20) erzeugter Synthesegasstrom (24), oder Mischstrom aus Kohlenstoffoxiden und Wasserstoff, oder methanhaltiger Gasstrom anschließend einer chemischen oder biotechnologischen Anlage (25) zugeführt wird.

11. Verfahren nach Anspruch 10, **dadurch gekennzeichnet, dass** der Synthesegasstrom (24), oder Mischstrom aus Kohlenstoffoxiden und Wasserstoff, oder methanhaltige Gasstrom in der chemischen oder biotechnologischen Anlage (25) zur Herstellung von Methanol, wenigstens einem Alkohol und/oder wenigstens einem anderen chemischen Wertprodukt verwendet wird.

12. Verfahren nach einem der Ansprüche 1 bis 11, **dadurch gekennzeichnet, dass** der der Einrichtung (20) zugeführte Wasserstoff durch Pyrolyse von Kohlenwasserstoffen, insbesondere Methan oder Erdgas erzeugt wird.

13. Verfahren nach Anspruch 12, **dadurch gekennzeichnet, dass** der bei der Pyrolyse von Kohlenwasserstoffen, insbesondere Methan oder Erdgas anfallende Pyrolysekohlenstoff zur Herstellung von Anoden für die elektrolytische Herstellung von Aluminium verwendet wird.

14. Verfahren nach einem der Ansprüche 1 bis 13, **dadurch gekennzeichnet, dass** in der Vorrichtung (10) zur Wärmeübertragung durch den Abgasstrom (9) ein methanhaltiger Gasstrom (15), insbesondere ein Einsatzgasstrom für eine Methanpyrolyse erwärmt wird.

15. Verfahren nach einem der Ansprüche 1 bis 14, **dadurch gekennzeichnet, dass** die bei der Herstellung der Anode entstehenden leichtflüchtigen Kohlenwasserstoffen über eine Leitung (27) in den Reaktor zur Pyrolyse der Kohlenwasserstoffe (21) zurückgeführt werden.

16. Anlagenverbund umfassend eine Elektrolysevorrichtung (7) zur Herstellung von Aluminium durch schmelzelektrolytische Reduktion von Aluminiumoxid unter Verwendung wenigstens einer Anode aus einem kohlenstoffhaltigen Material, wenigstens eine Einrichtung (16) zur Aufreinigung und/oder Konditionierung des Abgasstroms (12, 14) aus der Anlage (7) zur Herstellung von Aluminium, **dadurch gekennzeichnet, dass** der Anlagenverbund weiterhin wenigstens einen Reaktor zur Umsetzung des Abgasstroms mit Wasserstoff zu Synthesegas und/oder Methan und/oder eine Vorrichtung zum Mischen des Abgasstroms mit Wasserstoff für die nachfolgende Verwertung in einer chemische oder biotechnologische Anlage (25) zur Herstellung von Methanol, wenigstens einem Alkohol und/oder wenigstens einem anderen chemischen Wertprodukt umfasst.

17. Anlagenverbund nach Anspruch 16, **dadurch gekennzeichnet, dass** dieser wenigstens eine Vorrichtung (10) zur Wärmeübertragung umfasst, in der wenigstens ein Teilstrom (13) des Kohlenstoffoxide enthaltenden Abgasstroms (9) aus der Anlage (7) zur Herstellung von Aluminium auf eine geringere Temperatur abgekühlt wird.

18. Anlagenverbund nach Anspruch 16 oder 17 , **dadurch gekennzeichnet, dass** dieser weiterhin eine Einrichtung (20) umfasst, der die im Abgasstrom enthaltenen Kohlenstoffoxide zugeführt werden, in der eine reverse Wassergas-Shift-Reaktion durchgeführt wird, bei der mindestens ein Anteil des Kohlenstoffdioxids mit Wasserstoff umgesetzt und zu Kohlenstoffmonoxid reduziert und so ein Synthesegasstrom (24) erzeugt wird oder in der eine Sabatier-Reaktion durchgeführt wird, in der Kohlenstoffdioxid und/oder Kohlenstoffmonoxid durch Umsetzung mit Wasserstoff in Methan umgewandelt wird oder in der die im Abgasstrom enthaltenen Kohlenstoffoxide mit einem Wasserstoffstrom (23) gemischt werden.

19. Anlagenverbund nach einem der Ansprüche 16 bis 18, **dadurch gekennzeichnet, dass** dieser wenigstens zwei voneinander unabhängige Leitungen umfasst, wobei mittels der ersten Leitung ein in der Vorrichtung (10) zur Wärmeübertragung abgekühlter erster Abgasstrom (12) einerseits und davon unabhängig mittels der zweiten Leitung ein zweiter ungekühlter Abgasstrom (14) direkt aus der Anlage (7) zur Herstellung von Aluminium der Einrichtung (16) zur Aufreinigung und/oder Konditionierung zugeführt werden können.

## Claims

1. A process for the treatment of an offgas stream which is formed in a plant for the production of aluminum by electrolytic reduction of aluminum oxide in the melt using at least one anode composed of a carbon-comprising material and which owing to the reduction of the aluminum oxide by means of the carbon comprises carbon oxides, wherein at least a substream of the carbon oxides comprised in the offgas stream is purified and/or conditioned, reacted with hydrogen and reduced to carbon monoxide and/or methane or is mixed with a hydrogen stream and subsequently passed to utilization in a chemical or biotechnological reaction.

2. The process according to claim 1, wherein the carbon oxides comprised in the offgas stream are fed to a device (20) in which a reverse water gas shift reaction in which at least part of the carbon dioxide is reacted with hydrogen and reduced to carbon monoxide so as to produce a synthesis gas stream (24) is carried out.

3. The process according to claim 1, wherein the carbon oxides comprised in the offgas stream are fed to a device (20) in which a Sabatier reaction in which carbon dioxide and/or carbon monoxide are converted into methane by reaction with hydrogen is carried out.

4. The process according to claim 1, wherein the carbon oxides comprised in the offgas stream are fed to a device (20) in which they are mixed with a hydrogen stream (23).

5. The process according to any of claims 1 to 4, wherein at least a substream (14) of offgases from the plant (7) for producing aluminum is fed without prior cooling to the device (16) for purification and/or conditioning of the offgas.

6. The process according to any of claims 1 to 5, wherein at least a substream (12), in particular a second substream (12), of the offgas stream (11) is firstly cooled to a lower temperature in an apparatus (10) for heat exchange after leaving the plant for producing aluminum and is then fed to the device (16) for purification and/or conditioning of the offgas.

7. The process according to any of claims 1 to 6, wherein at least a first substream (13) of the offgas stream (9) comprising carbon oxides from the plant (7) for producing aluminum is recirculated to this plant.

8. The process according to claim 7, wherein the offgas stream (9) is firstly cooled to a lower temperature in an apparatus (10) for heat exchange after leaving the plant and at least a first substream (13) of the cooled offgas stream (11) is recirculated to the plant (7) for producing aluminum.

9. The process according to any of claims 1 to 8, wherein an offgas stream (9) comprising the carbon oxides from the plant (7) for producing aluminum is firstly cooled to a lower temperature in an apparatus (10) for heat exchange and this cooled gas stream is then divided into at least two substreams (12, 13) of which one substream (12) is fed to the device (16) for purification and/or conditioning of the offgas while a further substream (13) is recirculated to the plant (7) for producing aluminum.

10. The process according to any of claims 1 to 9, wherein a synthesis gas stream (24) produced in the device (20) or a mixed stream composed of carbon oxides and hydrogen or a methane-comprising gas stream is subsequently fed to a chemical or biotechnological plant (25) .

11. The process according to claim 10, wherein the synthesis gas stream (24) or the mixed stream composed of carbon oxides and hydrogen or the methane-comprising gas stream is used in the chemical or biotechnological plant (25) in order to produce methanol, at least one alcohol and/or at least one other chemical product of value.

12. The process according to any of claims 1 to 11, wherein the hydrogen fed to the device (20) is produced by pyrolysis of hydrocarbons, in particular methane or natural gas.

13. The process according to claim 12, wherein the pyrolysis carbon formed in the pyrolysis of hydrocarbons, in particular methane or natural gas, is used for producing anodes for the electrolytic production of aluminum.

14. The process according to any of claims 1 to 13, wherein a methane-comprising gas stream (15), in particular a feed gas stream for a methane pyrolysis, is heated by the offgas stream (9) in the apparatus (10) for heat exchange.

15. The process according to any of claims 1 to 14, wherein the volatile hydrocarbons formed in the production of the anode are recirculated via a conduit (27) to the reactor for the pyrolysis of the hydrocarbons (21) .

16. An integrated plant comprising an electrolysis apparatus (7) for the production of aluminum by melt-electrolytic reduction of aluminum oxide using at least one anode composed of a carbon-comprising material, at least one device (16) for purification and/or conditioning of the offgas stream (12, 14) from the plant (7) for producing aluminum, wherein the integrated plant further comprises at least one reactor for reaction of the offgas stream with hydrogen to form synthesis gas and/or to form methane and/or an apparatus for mixing the offgas stream with hydrogen for subsequent utilization in a chemical or biotechnological plant (25) to produce methanol, at least one alcohol and/or at least one other chemical product of value.

17. The integrated plant according to claim 16, wherein it comprises at least one apparatus (10) for heat exchange in which at least a substream (13) of the offgas stream (9) comprising carbon oxides from the plant (7) for producing aluminum is cooled to a lower temperature.

18. The integrated plant according to claim 16 or 17, wherein it further comprises a device (20) to which the carbon oxides comprised in the offgas stream are fed, in which a reverse water gas shift reaction in which at least part of the carbon dioxide is reacted with hydrogen and reduced to carbon monoxide so as to produce a synthesis gas stream (24) is carried out or in which a Sabatier reaction in which carbon dioxide and/or carbon monoxide are converted into methane by reaction with hydrogen is carried out or in which the carbon oxides comprised in the offgas stream are mixed with a hydrogen stream (23).

19. The integrated plant according to any of claims 16 to 18, wherein it comprises at least two independent conduits, where a first offgas stream (12) which has been cooled in the apparatus (10) for heat exchange can be conveyed by means of the first conduit and, independently thereof, a second uncooled offgas stream (14) can be conveyed by means of the second conduit directly from the plant (7) for producing aluminum into the device (16) for purification and/or conditioning.

## Revendications

1. Procédé pour le traitement d'un flux de gaz résiduaire produit dans une installation destinée à la production d'aluminium par réduction électrolytique d'oxyde d'aluminium en masse fondue, avec utilisation d'au moins une anode en un matériau contenant du carbone, lequel flux contient des oxydes de carbone en raison de la réduction de l'oxyde aluminium au moyen du carbone, **caractérisé en ce qu'**au moins un flux partiel des oxydes de carbone contenus dans le flux de gaz résiduaire est purifié et/ou conditionné, transformé avec de l'hydrogène et réduit en monoxyde de carbone et/ou en méthane ou mélangé avec un flux d'hydrogène et ensuite introduit pour une valorisation dans une réaction chimique ou biotechnologique.

2. Procédé selon la revendication 1, **caractérisé en ce que** les oxydes de carbone contenus dans le flux de gaz résiduaire sont introduits dans un dispositif (20) dans lequel est réalisée une réaction inverse de conversion de gaz à l'eau, lors de laquelle au moins une partie du dioxyde de carbone est transformée avec de l'hydrogène et réduite en monoxyde de carbone et un flux de gaz de synthèse (24) est ainsi formé.

3. Procédé selon la revendication 1, **caractérisé en ce que** les oxydes de carbone contenus dans le flux de gaz résiduaire sont introduits dans un dispositif (20) dans lequel est réalisée une réaction de Sabatier, dans laquelle du dioxyde de carbone et/ou du monoxyde de carbone est converti en méthane par transformation avec de l'hydrogène.

4. Procédé selon la revendication 1, **caractérisé en ce que** les oxydes de carbone contenus dans le flux de gaz résiduaire sont introduits dans un dispositif (20) dans lequel ils sont mélangés avec un flux d'hydrogène (23).

5. Procédé selon l'une quelconque des revendications 1 à 4, **caractérisé en ce qu'**au moins un flux partiel (14) de gaz résiduaires provenant de l'installation (7) destinée à la production d'aluminium est introduit sans refroidissement préalable dans le dispositif (16) destiné à la purification et/ou au conditionnement du gaz résiduaire.

6. Procédé selon l'une quelconque des revendications 1 à 5, **caractérisé en ce qu'**au moins un flux partiel (12), en particulier un deuxième flux partiel (12), du flux de gaz résiduaire (11), après avoir quitté l'installation destinée à la production d'aluminium, est d'abord refroidi à une température inférieure dans un appareil (10) destiné au transfert de chaleur et ensuite introduit dans le dispositif (16) destiné à la purification et/ou au conditionnement du gaz résiduaire.

7. Procédé selon l'une quelconque des revendications 1 à 6, **caractérisé en ce qu'**au moins un premier flux partiel (13) du flux de gaz résiduaire (9) contenant des oxydes de carbone provenant de l'installation (7) destinée à la production d'aluminium est recyclé dans cette installation.

8. Procédé selon la revendication 7, caractérisé en ce le flux de gaz résiduaire (9), après avoir quitté l'installation, est d'abord refroidi à une température inférieure dans un appareil (10) destiné au transfert de chaleur et au moins un premier flux partiel (13) du flux de gaz résiduaire (11) refroidi est recyclé dans l'installation (7) destinée à la production d'aluminium.

9. Procédé selon l'une quelconque des revendications 1 à 8, **caractérisé en ce qu'**un flux de gaz résiduaire (9) contenant les oxydes de carbone provenant de l'installation (7) destinée à la production d'aluminium est d'abord refroidi à une température inférieure dans un appareil (10) destiné au transfert de chaleur et ce flux de gaz refroidi est ensuite réparti en au moins deux flux partiels (12, 13), dont un flux partiel (12) est introduit dans le dispositif (16) destiné à la purification et/ou au conditionnement du gaz résiduaire alors qu'un autre flux partiel (13) est recyclé dans l'installation (7) destinée à la production d'aluminium.

10. Procédé selon l'une quelconque des revendications 1 à 9, **caractérisé en ce qu'**un flux de gaz de synthèse (24), ou un flux mixte d'oxydes de carbone et d'hydrogène ou un flux de gaz contenant du méthane, formé dans un dispositif (20) est ensuite introduit dans une installation chimique ou biotechnologique (25).

11. Procédé selon la revendication 10, **caractérisé en ce qu'**un flux de gaz de synthèse (24), ou un flux mixte d'oxydes de carbone et d'hydrogène ou un flux de gaz contenant du méthane dans l'installation chimique ou biotechnologique (25) est utilisé pour la préparation de méthanol, d'au moins un alcool et/ou d'au moins un autre produit de valeur chimique.

12. Procédé selon l'une quelconque des revendications 1 à 11, **caractérisé en ce que** l'hydrogène introduit dans le dispositif (20) est formé par pyrolyse d'hydrocarbures, en particulier de méthane ou de gaz naturel.

13. Procédé selon la revendication 12, **caractérisé en ce que** le carbone de pyrolyse produit lors de la pyrolyse d'hydrocarbures, en particulier de méthane ou de gaz naturel, est utilisé pour la production d'anodes pour la production électrolytique d'aluminium.

14. Procédé selon l'une quelconque des revendications 1 à 13, **caractérisé en ce qu'**un flux de gaz contenant du méthane (15), en particulier un flux de gaz d'utilisation pour une pyrolyse de méthane, est chauffé par le flux de gaz résiduaire (9) dans l'appareil (10) destiné au transfert de chaleur.

15. Procédé selon l'une quelconque des revendications 1 à 14, **caractérisé en ce que** les hydrocarbures volatils formés lors de la production de l'anode sont recyclés par l'intermédiaire d'une conduite (27) dans le réacteur destiné à la pyrolyse des hydrocarbures (21).

16. Ensemble d'installations comprenant un appareil d'électrolyse (7) destiné à la production d'aluminium par réduction électrolytique en masse fondue d'oxyde d'aluminium avec utilisation d'au moins une anode en un matériau contenant du carbone, au moins un dispositif (16) destiné à la purification et/ou au conditionnement du flux de gaz résiduaire (12, 14) provenant de l'installation (7) destinée à la production d'aluminium, **caractérisé en ce que** l'ensemble des installations comprend en outre au moins un réacteur destiné à la transformation du flux de gaz résiduaire avec de l'hydrogène en gaz de synthèse et/ou en méthane et/ou un appareil pour le mélange du flux de gaz résiduaire avec de l'hydrogène pour la valorisation consécutive dans une installation chimique ou biotechnologique (25) destinée à la préparation de méthanol, d'au moins un alcool et/ou d'au moins un autre produit de valeur chimique.

17. Ensemble d'installations selon la revendication 16, **caractérisé en ce que** celui-ci comprend au moins un appareil (10) destiné au transfert de chaleur, dans lequel au moins un flux partiel (13) du flux de gaz résiduaire (9) contenant des oxydes de carbone provenant de l'installation (7) destinée à la production d'aluminium est refroidi à une température inférieure.

18. Ensemble d'installations selon la revendication 16 ou 17, **caractérisé en ce que** celui-ci comprend en outre un dispositif (20) qui est alimenté en oxydes de carbone contenus dans le flux de gaz résiduaire, dans lequel dispositif est réalisée une réaction inverse de conversion de gaz à l'eau, lors de laquelle au moins une partie du dioxyde de carbone est transformée avec de l'hydrogène et réduite en monoxyde de carbone et un flux de gaz de synthèse (24) est ainsi formé ou dans lequel dispositif est réalisée une réaction de Sabatier, dans laquelle du dioxyde de carbone et/ou du monoxyde de carbone est converti en méthane par transformation avec de l'hydrogène ou dans lequel les oxydes de carbone contenus dans le flux de gaz résiduaire sont mélangés avec un flux d'hydrogène (23).

19. Ensemble d'installations selon l'une quelconque des revendications 16 à 18, **caractérisé en ce que** celui-ci comprend au moins deux conduites indépendantes l'une de l'autre, la première conduite permettant d'une part de recycler un premier flux de gaz résiduaire (12) refroidi dans un appareil (10) destiné au transfert de chaleur et, indépendamment, la deuxième conduite permettant de recycler un deuxième flux de gaz résiduaire (14) non refroidi, directement à partir de l'installation (7) destinée à la production d'aluminium dans le dispositif (16) destiné à la purification et/ou au conditionnement.
